(19) Europäisches Patentamt
European Patent Office
Office européen des brevets

(11) **EP 1 598 091 B1**

(12) **EUROPEAN PATENT SPECIFICATION**

(45) Date of publication and mention
of the grant of the patent:
**21.06.2006 Bulletin 2006/25**

(51) Int Cl.:
*A61N 1/32* (2006.01)     *A61N 1/38* (2006.01)

(21) Application number: **04252993.3**

(22) Date of filing: **21.05.2004**

(54) **Non-specified oscillatory wave signal generating method and apparatus**

Verfahren und Gerät zur Erzeugung eines unspezifizierten Oszillator-Wellensignals

Procède et dispositif pour générer un signal non spécifié d'onde oscillatoire

(84) Designated Contracting States:
**AT BE BG CH CY CZ DE DK EE ES FI FR GB GR HU IE IT LI LU MC NL PL PT RO SE SI SK TR**
Designated Extension States:
**AL HR LT LV MK**

(43) Date of publication of application:
**23.11.2005 Bulletin 2005/47**

(73) Proprietor: **Xie Neng Biotech Corporation**
**Ling-Ya District,**
**Kaohsiung City (TW)**

(72) Inventor: **Liang, Wen-Lin**
**Kachsiung Hsien**
**Taiwan (CN)**

(74) Representative: **Mounteney, Simon James**
**Marks & Clerk**
**90 Long Acre**
**London WC2E 9RA (GB)**

(56) References cited:
**WO-A-90/11043**          **US-A- 4 338 945**

- **OLMES A ET AL: "An improved method of measuring laser induced pressure transients" APPLIED PHYSICS B (LASERS AND OPTICS) SPRINGER-VERLAG GERMANY, vol. B64, no. 6, 1997, pages 677-682, XP002299170 ISSN: 0946-2171**

## Description

The invention relates to the generation of oscillatory wave signals, more particularly to a method and apparatus for generating a non-fixed oscillatory wave signal that is suitable for electrotherapeutic applications.

In conventional electrotherapy, a specific oscillatory wave signal is applied to a human body for physical therapy. However, since the human body is able to adapt to the stimulus of the specific oscillatory wave signal after a period of exposure to the same, the desired therapeutic effect cannot be ensured.

Therefore, the object of the present invention is to provide a method and apparatus for generating a non-fixed oscillatory wave signal that is suitable for application to a patient undergoing electrotherapy.

According to one aspect of the present invention, there is provided a method of generating an oscillatory wave signal. The method comprises the steps of:

a) establishing a set of digitized time-domain basic wave signals;
b) transforming the digitized time-domain basic wave signals into a corresponding set of frequency spectra;
c) randomly processing the set of frequency spectra;
d) combining the set of frequency spectra processed in step c) so as to obtain a mixed spectrum;
e) transforming the mixed spectrum into a time-domain synthesized wave signal corresponding to the mixed spectrum; and
f) converting the synthesized wave signal into an analog wave signal.

According to another aspect of the present invention, there is provided an apparatus for generating an oscillatory wave signal. The apparatus comprises:

a signal generating module for establishing a set of digitized time-domain basic wave signals;
a first transforming unit coupled to the signal generating module for transforming the digitized time-domain basic wave signals into a corresponding set of frequency spectra;
a processing unit coupled to the first transforming module for randomly processing the set of frequency spectra;
a combining module coupled to the processing unit for combining the set of frequency spectra processed by the processing unit so as to obtain a mixed spectrum;
a second transforming unit coupled to the combining module for transforming the mixed spectrum into a time-domain synthesized wave signal corresponding to the mixed spectrum; and
an output module coupled to the second transforming unit for converting the synthesized wave signal into an analog wave signal and for outputting the analog wave signal.

Other features and advantages of the present invention will become apparent in the following detailed description of the preferred embodiment with reference to the accompanying drawings, of which:

Figure 1 is a schematic block diagram illustrating an apparatus for implementing the preferred embodiment of a method of generating an oscillatory wave signal according to the present invention;
Figure 2 is a flow chart illustrating how the apparatus of Figure 1 generates an oscillatory wave signal in accordance with the method of the preferred embodiment;
Figures 3a to 3c are plots of exemplary digitized time-domain basic wave signals (XG, XH, XM) established in the preferred embodiment;
Figure 4a to 4c are plots of frequency spectra transformed from the digitized time-domain basic wave signals (XG, XH, XM) of Figures 3a to 3c;
Figure 5 is a chart to illustrate how the frequency spectra of Figures 4a to 4c are combined in accordance with the preferred embodiment; and
Figure 6 is a plot of a time-domain synthesized wave signal resulting from the processing of the digitized time-domain basic wave signals (XG, XH, XM) in accordance with the preferred embodiment.

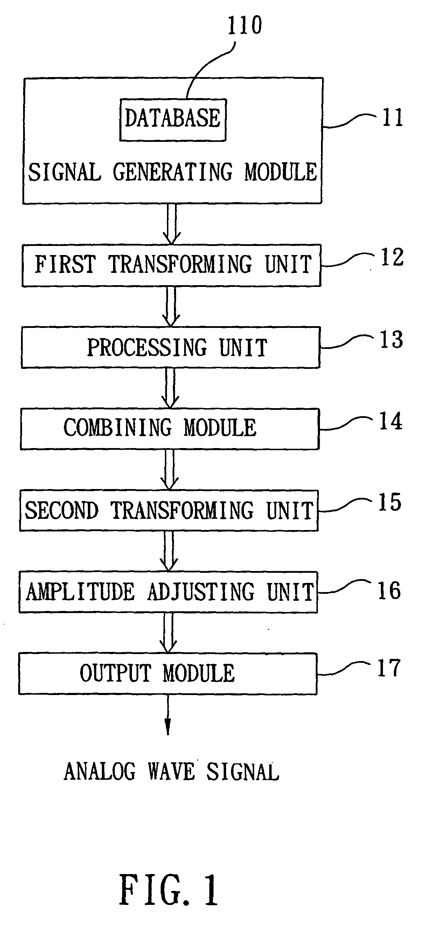

Figure 1 illustrates an apparatus configuration for implementing the preferred embodiment of a method of generating a non-fixed oscillatory wave signal according to the present invention. The apparatus includes a signal generating module 11, a first transforming unit 12, a processing unit 13, a combining module 14, a second transforming unit 15, an amplitude adjusting unit 16, and an output module 17.

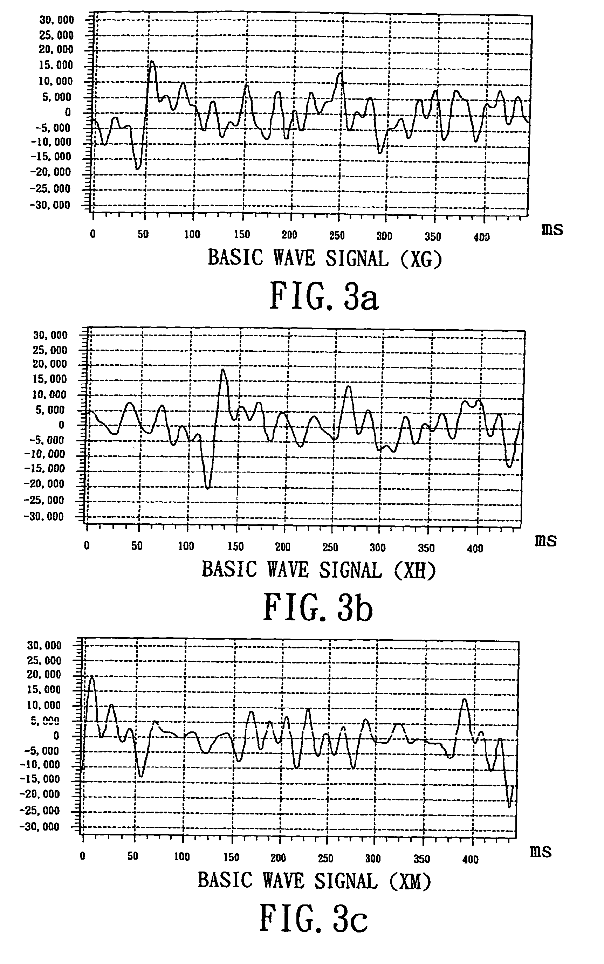

The signal generating module 11 is operable so as to establish a set of digitized time-domain basic wave signals in a trial-and-error manner. In this embodiment, the set of digitized time-domain basic wave signals, which can be stored in a database 110 built-in the signal generating modules 11, includes three digitized time-domain basic wave signals (XG, XH, XM) that are suitable for application to a patient undergoing electrotherapy, as shown in Figures 3a to 3c.

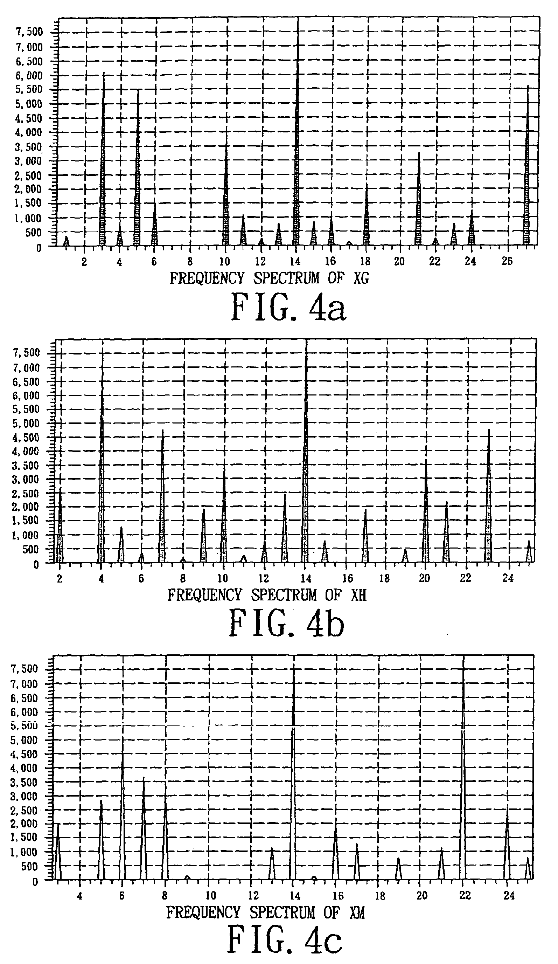

The first transforming unit 12 is coupled to the signal generating module 11, and is operable so as to transform the digitized time-domain basic wave signals (XG, XH, XM) into corresponding frequency spectra, as shown in Figures 4a to 4c. In this embodiment, the first transforming unit 12 performs Fast Fourier Transform to obtain the frequency spectra. Each of the frequency spectra includes a baseband spectral component and a plurality of harmonic spectral components. The frequencies of the harmonic spectral components are determined according to the following Equation 1:

$$f_n = f_0 + f_i \times n \qquad ...(Equation\ 1)$$

where $f_0$ denotes the frequency of the baseband spectral component, $f_n$ denotes the frequency of the $n^{th}$ harmonic spectral component, and $f_i$ denotes the frequency difference between the baseband spectral component and the first harmonic spectral component or between adj acent ones of the harmonic spectral components, where "n" is a natural number, and where $f_n$, $f_0$ and $f_i$ are positive real numbers.

The processing unit 13 is coupled to the first transforming module 12, and is operable so as to randomly process the set of frequency spectra. In this embodiment, the processing unit 13 randomly processes the frequency spectra by multiplying a frequency scale of each of the frequency spectra by a random natural number chosen independently of those used to process other ones of the frequency spectra. In this example, the random natural number for each of the frequency spectra is equal to 1.

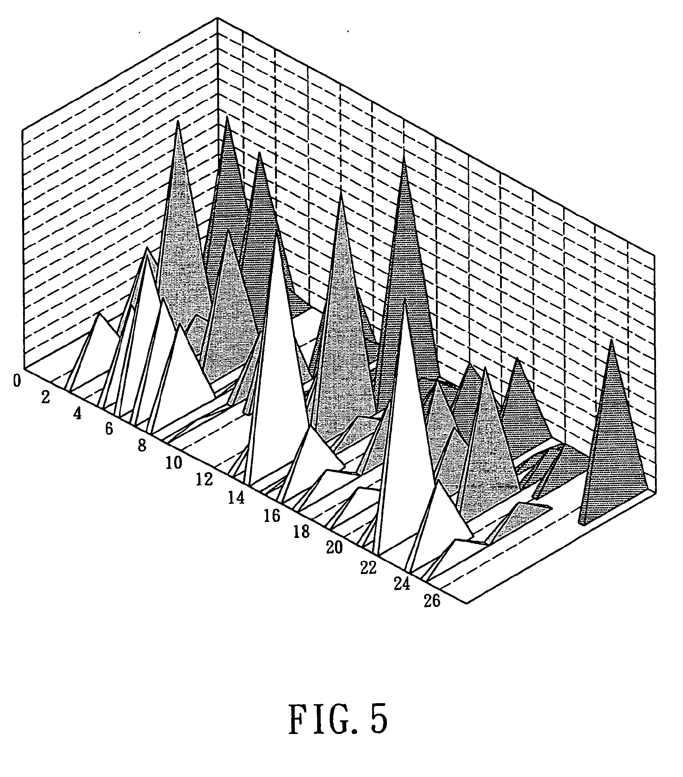

The combining module 14 is coupled to the processing unit 13 and is operable so as to combine the frequency spectra processed by the processing unit 13, thereby resulting in a mixed spectrum, as shown in Figure 5.

The second transforming unit 15 is coupled to the combining module 14, and is operable so as to transform the mixed spectrum into a time-domain synthesized wave signal. In this embodiment, the second transforming unit 15 performs Inverse Fast Fourier Transform to obtain the synthesized wave signal.

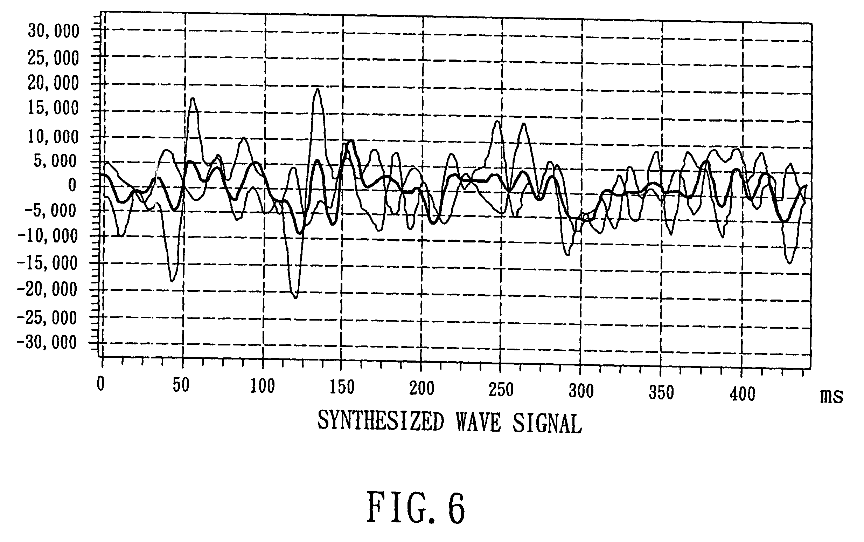

The amplitude adjusting unit 16 is coupled to the second transforming unit 15, and is operable so as to adjust amplitudes of the synthesized wave signal such that the latter is suitable for application to a human body, as shown in Figure 6. In this embodiment, the amplitude adjusting unit 16 divides each of the amplitudes of the synthesized wave signal by an average of the amplitudes of the synthesized wave signal.

The output module 17 is coupled to the amplitude adjusting unit 16, and is operable so as to convert the synthesized wave signal adjusted by the amplitude adjusting unit 16 into an analog wave signal and so as to output the analog wave signal in the form of small current and low voltage via an electrode (not shown). In this embodiment, the analog wave signal outputted by the output module 17 is the non-fixed oscillatory wave signal, and is suitable for application to a patient undergoing electrotherapy.

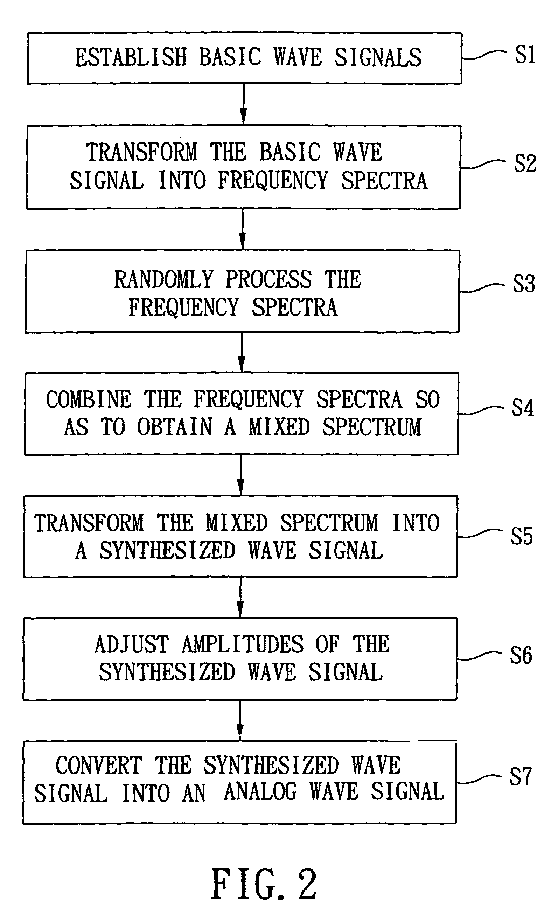

Referring to Figure 2, there is shown a flow chart to illustrate how the apparatus generates the non-fixed oscillatory wave signal in accordance with the method of the preferred embodiment. In step S1, the signal generating module 11 is operable so as to establishe the set of digitized time-domain basic wave signals (XG, XH, XM). In step S2, the first transforming unit 12 transforms the digitized time-domain basic wave signals (XG, XH, XM) into the corresponding set of frequency spectra. In step S3, the processing unit 13 randomly processes the set of frequency spectra. In step S4, the combining unit 14 combines the set of frequency spectra processed by the processing unit 13 so as to obtain the mixed spectrum. In step S5, the second transforming unit 15 transforms the mixed spectrum into a time-domain synthesized wave signal corresponding to the mixed spectrum. In step S6, the amplitude adjusting unit 16 adjusts the amplitudes of the synthesized wave signal so as to be suitable for application to a human body. In step S7, the output module 17 converts the synthesized wave signal adjusted by the amplitude adjusting unit 16 into the analog wave signal (i.e., the non-fixed oscillatory wave signal that is suitable for a patient undergoing electrotherapy).

To sum up, this invention discloses the generation of non-fixed oscillatory wave signals for application to a patient undergoing electrotherapy. Because of the non-fixed characteristics of the oscillatory wave signal, the human body is unable to adapt to its stimulus after a period of exposure to the same, thereby ensuring the desired therapeutic effect.

**Claims**

1. A method of generating an oscillatory wave signal, **characterized by** the steps of:

 a) establishing a set of digitized time-domain basic wave signals;
 b) transforming the digitized time-domain basic wave signals into a corresponding set of frequency spectra;
 c) randomly processing the set of frequency spectra;
 d) combining the set of frequency spectra processed in step c) so as to obtain a mixed spectrum;

e) transforming the mixed spectrum into a time-domain synthesized wave signal corresponding to the mixed spectrum; and

f) converting the synthesized wave signal into an analog wave signal.

2. The method as claimed in Claim 1, **characterized in that** the digitized time-domain basic wave signals are suitable for application to a patient undergoing electrotherapy.

3. The method as claimed in Claim 1, **characterized in that** the analog wave signal generated in step f) is the oscillatory wave signal and is suitable for application to a patient undergoing electrotherapy.

4. The method as claimed in Claim 1, **characterized in that** the set of frequency spectra are randomly processed in step c) by multiplying a frequency scale of each of the set of frequency spectra by a random natural number chosen independently of those used to process other ones of the frequency spectra.

5. The method as claimed in Claim 2, further **characterized by**, prior to step f), the step of e') adjusting amplitudes of the synthesized wave signal so as to be suitable for application to a human body.

6. The method as claimed in Claim 5, further **characterized in that** in step e'), each of the amplitudes of the synthesized wave signal is divided by an average of the amplitudes of the synthesized wave signal.

7. The method as claimed in Claim 1, **characterized in that** step b) is performed using Fast Fourier Transform.

8. The method as claimed in Claim 1, **characterized in that** step e) is performed using Inverse Fast Fourier Transform.

9. An apparatus for generating an oscillatorywave signal, **characterized by**:

a signal generating module (11) for establishing a set of digitized time-domain basic wave signals;

a first transforming unit (12) coupled to said signal generating module (11) for transforming the digitized time-domain basic wave signals into a corresponding set of frequency spectra;

a processing unit (13) coupled to said first transforming module (12) for randomly processing the set of frequency spectra;

a combining module (14) coupled to said processing unit (13) for combining the set of frequency spectra processed by said processing unit so as to obtain a mixed spectrum;

a second transforming unit (15) coupled to said combining module (14) for transforming the mixed spectrum into a time-domain synthesized wave signal corresponding to the mixed spectrum; and

an output module (17) coupled to said second transforming unit (15) for converting the synthesized wave signal into an analog wave signal and for outputting the analog wave signal.

10. The apparatus as claimed in Claim 9, **characterized in that** the digitized time-domain basic wave signals are suitable for application to a patient undergoing electrotherapy.

11. The apparatus as claimed in Claim 9, **characterized in that** the analog wave signal outputted by said output module (17) is the oscillatory wave signal and is suitable for application to a patient undergoing electrotherapy.

12. The apparatus as claimed in Claim 9, **characterized in that** said processing unit (13) randomly processes the set of frequency spectra are by multiplying a frequency scale of each of the set of frequency spectra by a random natural number chosen independently of those used to process other ones of the frequency spectra.

13. The apparatus as claimed in Claim 10, further **characterized by** an amplitude adjusting unit (16) coupled to said second transforming unit (15) and said output module (17) for adjusting amplitudes of the synthesized wave signal so that the synthesized wave signal is suitable for application to a human body.

14. The apparatus as claimed in Claim 13, further **characterized in that** said amplitude adjusting unit (16) divides each of the amplitudes of the synthesized wave signal by an average of the amplitudes of the synthesized wave signal.

15. The apparatus as claimed in Claim 9, **characterized in that** said first transforming unit (12) performs Fast Fourier Transform to obtain the set of frequency spectra.

**16.** The apparatus as claimed in Claim 9, **characterized in that** said second transforming unit (15) performs Inverse Fast Fourier Transform to obtain the synthesized wave signal.

**Patentansprüche**

**1.** Verfahren zur Erzeugung eines Schwingungswellensignals, **gekennzeichnet durch** die folgenden Schritte:

  a) Aufbauen einer Menge von digitalisierten Zeitbereichs-Basiswellensignale;
  b) Umwandeln der digitalisierten Zeitbereichs-Basiswellensignale in eine entsprechende Menge von Frequenzspektren;
  c) zufälliges Verarbeiten der Menge von Frequenzspektren;
  d) Kombinieren der in Schritt c) verarbeiteten Menge von Frequenzspektren, um ein gemischtes Spektrum zu erhalten;
  e) Umwandeln des gemischten Spektrums in ein synthetisiertes Zeitbereichs-Wellensignal, das dem gemischten Spektrum entspricht; und
  f) Konvertieren des synthetisierten Wellensignals in ein analoges Wellensignal.

**2.** Verfahren nach Anspruch 1, **dadurch gekennzeichnet, daß** die digitalisierten Zeitbereichs-Basiswellensignale zur Anwendung bei einem Patienten, der sich einer Elektrotherapie unterzieht, geeignet sind.

**3.** Verfahren nach Anspruch 1, **dadurch gekennzeichnet, daß** das in Schritt f) erzeugte analoge Wellensignal das Schwingungswellensignal ist und zur Anwendung bei einem Patienten, der sich einer Elektrotherapie unterzieht, geeignet ist.

**4.** Verfahren nach Anspruch 1, **dadurch gekennzeichnet, daß** die Menge von Frequenzspektren in Schritt c) zufällig verarbeitet wird, indem eine Frequenzskala jedes aus der Menge von Frequenzspektren mit einer natürlichen Zufallszahl multipliziert wird, die unabhängig von denen gewählt wird, die zur Verarbeitung anderer der Frequenzspektren verwendet werden.

**5.** Verfahren nach Anspruch 2, ferner **gekennzeichnet durch** den folgenden vor Schritt f) erfolgenden Schritt: e') Regulieren der Amplituden des synthetisierten Wellensignals, so daß es zur Anwendung am menschlichen Körper geeignet ist.

**6.** Verfahren nach Anspruch 5, ferner **dadurch gekennzeichnet, daß** in Schritt e') jede der Amplituden des synthetisierten Wellensignals durch einen Mittelwert der Amplituden des synthetisierten Wellensignals dividiert wird.

**7.** Verfahren nach Anspruch 1, **dadurch gekennzeichnet, daß** Schritt b) unter Verwendung der schnellen Fourier-Transformation durchgeführt wird.

**8.** Verfahren nach Anspruch 1, **dadurch gekennzeichnet, daß** Schritt e) unter Verwendung der inversen schnellen Fourier-Transformation durchgeführt wird.

**9.** Vorrichtung zur Erzeugung eines Schwingungswellensignals, **gekennzeichnet durch**:

  ein Signalerzeugungsmodul (11) zum Aufbauen einer Menge von digitalisierten Zeitbereichs-Basiswellensignale;
  eine mit dem Signalerzeugungsmodul (11) gekoppelte erste Umwandlungseinheit (12) zum Umwandeln der digitalisierten Zeitbereichs-Basiswellensignale in eine entsprechende Menge von Frequenzspektren;
  eine mit der ersten Umwandlungseinheit (12) gekoppelte Verarbeitungseinheit (13) zum zufälligen Verarbeiten der Menge von Frequenzspektren;
  ein mit der Verarbeitungseinheit (13) gekoppeltes Kombiniermodul (14) zum Kombinieren der **durch** die Verarbeitungseinheit verarbeiteten Menge von Frequenzspektren, um ein gemischtes Spektrum zu erhalten;
  eine mit dem Kombiniermodul (14) gekoppelte zweite Umwandlungseinheit (15) zum Umwandeln des gemischten Spektrums in ein synthetisiertes Zeitbereichs-Wellensignal, das dem gemischten Spektrum entspricht; und
  ein mit der zweiten Umwandlungseinheit (15) gekoppeltes Ausgabemodul (17) zum Konvertieren des synthetisierten Wellensignals in ein analoges Wellensignal und zum Ausgeben des analogen Wellensignals.

**10.** Vorrichtung nach Anspruch 9, **dadurch gekennzeichnet, daß** die digitalisierten Zeitbereichs-Basiswellensignale zur Anwendung bei einem Patienten, der sich einer Elektrotherapie unterzieht, geeignet sind.

**11.** Vorrichtung nach Anspruch 9, **dadurch gekennzeichnet, daß** das durch das Ausgabemodul (17) ausgegebene analoge Wellensignal das Schwingungswellensignal ist und zur Anwendung bei einem Patienten, der sich einer Elektrotherapie unterzieht, geeignet ist.

**12.** Vorrichtung nach Anspruch 9, **dadurch gekennzeichnet, daß** die Verarbeitungseinheit (13) die Menge von Frequenzspektren zufällig verarbeitet, indem eine Frequenzskala jedes aus der Menge von Frequenzspektren mit einer natürlichen Zufallszahl multipliziert wird, die unabhängig von denen gewählt wird, die zur Verarbeitung anderer der Frequenzspektren verwendet werden.

**13.** Vorrichtung nach Anspruch 10, ferner **gekennzeichnet durch** eine mit der zweiten Umwandlungseinheit (15) und dem Ausgabemodul (17) gekoppelte Amplitudenregulierungseinheit (16) zum Regulieren der Amplituden des synthetisierten Wellensignals, so daß es zur Anwendung am menschlichen Körper geeignet ist.

**14.** Vorrichtung nach Anspruch 13, ferner **dadurch gekennzeichnet, daß** die Amplitudenregulierungseinheit (16) jede der Amplituden des synthetisierten Wellensignals durch einen Mittelwert der Amplituden des synthetisierten Wellensignals dividiert.

**15.** Vorrichtung nach Anspruch 9, **dadurch gekennzeichnet, daß** die erste Umwandlungseinheit (12) eine schnelle Fourier-Transformation durchführt, um die Menge von Frequenzspektren zu erhalten.

**16.** Vorrichtung nach Anspruch 9, **dadurch gekennzeichnet, daß** die zweite Umwandlungseinheit (15) eine inverse schnelle Fourier-Transformation durchführt, um das synthetisierte Wellensignal zu erhalten.

## Revendications

**1.** Procédé de génération d'un signal d'onde oscillatoire, **caractérisé par** les étapes de :

a) établissement d'un jeu de signaux d'onde de base du domaine temporel numérisés ;
b) transformation des signaux d'onde de base du domaine temporel numérisés selon un jeu correspondant de spectres de fréquences ;
c) traitement de façon aléatoire du jeu de spectres de fréquences ;
d) combinaison du jeu de spectres de fréquences traité au niveau de l'étape c) afin d'obtenir un spectre mixte ;
e) transformation du spectre mixte selon un signal d'onde synthétisé du domaine temporel correspondant au spectre mixte ; et
f) conversion du signal d'onde synthétisé en un signal d'onde analogique.

**2.** Procédé selon la revendication 1, **caractérisé en ce que** les signaux d'onde de base du domaine temporel numérisés conviennent pour une application sur un patient subissant une électrothérapie.

**3.** Procédé selon la revendication 1, **caractérisé en ce que** le signal d'onde analogique généré au niveau de l'étape f) est le signal d'onde oscillatoire et il convient pour une application sur un patient subissant une électrothérapie.

**4.** Procédé selon la revendication 1, **caractérisé en ce que** le jeu de spectres de fréquences est traité de façon aléatoire au niveau de l'étape c) en multipliant une échelle de fréquence de chaque spectre du jeu de spectres de fréquences par un nombre naturel aléatoire choisi indépendamment de ceux qui sont utilisés pour traiter d'autres spectres des spectres de fréquences.

**5.** Procédé selon la revendication 2, **caractérisé en outre par**, avant l'étape f), l'étape e') consistant à régler des amplitudes du signal d'onde synthétisé de manière à ce qu'elles conviennent pour une application sur un corps humain.

**6.** Procédé selon la revendication 5, **caractérisé en outre en ce que**, au niveau de l'étape e'), chacune des amplitudes du signal d'onde synthétisé est divisée par une moyenne des amplitudes du signal d'onde synthétisé.

**7.** Procédé selon la revendication 1, **caractérisé en ce que** l'étape b) est réalisée en utilisant une transformation rapide de Fourier.

**8.** Procédé selon la revendication 1, **caractérisé en ce que** l'étape e) est réalisée en utilisant une transformation rapide inverse de Fourier.

**9.** Appareil pour générer un signal d'onde oscillatoire, **caractérisé par** :

un module de génération de signal (11) pour établir un jeu de signaux d'onde de base du domaine temporel numérisés ;
une première unité de transformation (12) qui est couplée audit module de génération de signal (11) pour transformer les signaux d'onde de base du domaine temporel numérisés en un jeu correspondant de spectres de fréquences ;
une unité de traitement (13) qui est couplée audit premier module de transformation (12) pour traiter de façon aléatoire le jeu de spectres de fréquences ;
un module de combinaison (14) qui est couplé à ladite unité de traitement (13) pour combiner le jeu de spectres de fréquences traité par ladite unité de traitement de manière à obtenir un spectre mixte ;
une seconde unité de transformation (15) qui est couplée audit module de combinaison (14) pour transformer le spectre mixte en un signal d'onde synthétisé du domaine temporel correspondant au spectre mixte ; et
un module de sortie (17) qui est couplé à ladite seconde unité de transformation (15) pour convertir le signal d'onde synthétisé en un signal d'onde analogique et pour émettre en sortie le signal d'onde analogique.

**10.** Appareil selon la revendication 9, **caractérisé en ce que** les signaux d'onde de base du domaine temporel numérisés conviennent pour une application sur un patient qui subit une électrothérapie.

**11.** Appareil selon la revendication 9, **caractérisé en ce que** le signal d'onde analogique qui est émis en sortie par ledit module de sortie (17) est le signal d'onde oscillatoire et il convient pour une application sur un patient qui subit une électrothérapie.

**12.** Appareil selon la revendication 9, **caractérisé en ce que** ladite unité de traitement (13) traite de façon aléatoire le jeu de spectres de fréquences en multipliant une échelle de fréquence de chaque spectre du jeu de spectres de fréquences par un nombre naturel aléatoire qui est choisi indépendamment de ceux utilisés pour traiter d'autres spectres des spectres de fréquences.

**13.** Appareil selon la revendication 10, **caractérisé en outre par** une unité de réglage d'amplitude (16) qui est couplée à ladite seconde unité de transformation (15) et audit module de sortie (17) pour régler des amplitudes du signal d'onde synthétisé de telle sorte que le signal d'onde synthétisé convienne pour une application sur un corps humain.

**14.** Appareil selon la revendication 13, **caractérisé en outre en ce que** ladite unité de réglage d'amplitude (16) divise chacune des amplitudes du signal d'onde synthétisé par une moyenne des amplitudes du signal d'onde synthétisé.

**15.** Appareil selon la revendication 9, **caractérisé en ce que** ladite première unité de transformation (12) réalise une transformation rapide de Fourier pour obtenir le jeu de spectres de fréquences.

**16.** Appareil selon la revendication 9, **caractérisé en ce que** ladite seconde unité de transformation (15) réalise une transformation rapide inverse de Fourier pour obtenir le signal d'onde synthétisé.

110

DATABASE

SIGNAL GENERATING MODULE — 11

↓

FIRST TRANSFORMING UNIT — 12

↓

PROCESSING UNIT — 13

↓

COMBINING MODULE — 14

↓

SECOND TRANSFORMING UNIT — 15

↓

AMPLITUDE ADJUSTING UNIT — 16

↓

OUTPUT MODULE — 17

↓

ANALOG WAVE SIGNAL

FIG. 1

ESTABLISH BASIC WAVE SIGNALS — S1

TRANSFORM THE BASIC WAVE
SIGNAL INTO FREQUENCY SPECTRA — S2

RANDOMLY PROCESS THE
FREQUENCY SPECTRA — S3

COMBINE THE FREQUENCY SPECTRA SO
AS TO OBTAIN A MIXED SPECTRUM — S4

TRANSFORM THE MIXED SPECTRUM INTO
A SYNTHESIZED WAVE SIGNAL — S5

ADJUST AMPLITUDES OF THE
SYNTHESIZED WAVE SIGNAL — S6

CONVERT THE SYNTHESIZED WAVE
SIGNAL INTO AN ANALOG WAVE SIGNAL — S7

# FIG. 2

BASIC WAVE SIGNAL (XG)

FIG. 3a

BASIC WAVE SIGNAL (XH)

FIG. 3b

BASIC WAVE SIGNAL (XM)

FIG. 3c

FREQUENCY SPECTRUM OF XG

FIG. 4a

FREQUENCY SPECTRUM OF XH

FIG. 4b

FREQUENCY SPECTRUM OF XM

FIG. 4c

FIG. 5

SYNTHESIZED WAVE SIGNAL

FIG. 6

EP 1 598 091 B1